# EUROPEAN PATENT SPECIFICATION

(11) **EP 4 433 154 B1**
(45) Date of publication and mention of the grant of the patent: **22.10.2025**
(21) Application number: 22821856.6
(22) Date of filing: 10.11.2022
(51) Int. Cl.: A61N 1/372, A61N 1/375, A61N 1/39

(54) **IMPLANTABLE THERAPY SYSTEM FOR AN ANTIBRADYCARDIA AND ANTITACHYCARDIA STIMULATION**
IMPLANTIERBARES THERAPIESYSTEM FÜR EINE ANTIBRADYKARDIE- UND ANTITACHYKARDIE-STIMULATION
SYSTÈME THÉRAPEUTIQUE IMPLANTABLE POUR UNE STIMULATION ANTIBRADYCARDIQUE ET ANTITACHYCARDIQUE

(30) Priority: 19.11.2021 EP 21209243
(43) Date of publication of application: 25.09.2024
(73) Proprietor: BIOTRONIK SE & Co. KG, 12359 Berlin (DE)
(72) Inventor: DÖRR, Thomas, 12437 Berlin (DE); WEISS, Ingo, 12435 Berlin (DE)
(74) Representative: Biotronik Corporate Services SE
(86) International application number: PCT/EP2022/081494
(87) International publication number: WO 2023/088780

(56) References cited:
- EP-A1- 3 348 306
- WO-A1-2016/118847
- US-A1- 2015 142 069
- US-A1- 2017 216 611
- US-A1- 2017 312 514

## Description

The instant invention generally relates to an implantable therapy system for an antibradycardia and antitachycardia stimulation.

An implantable therapy system for an antibradycardia and antitachycardia stimulation generally comprises an implantable pacemaker device for emitting a cardiac stimulation signal for achieving an intracardiac pacing, and an implantable non-transvenous defibrillator device for emitting a shock pulse for achieving a defibrillation.

An implantable pacemaker may for example be subcutaneously implanted in a patient and may comprise leads carrying electrodes and extending from a generator unit of the pacemaker device into the patient's heart for example to provide a pacing action in the right ventricle of the heart. Alternatively, an implantable pacemaker device may be designed as a leadless pacemaker not comprising leads, but being directly implanted into the patient's heart, for example in the right ventricle in order to provide for a pacing action.

An implantable non-transvenous defibrillator device, also denoted as implantable non-transvenous cardioverter defibrillator (in short non-transvenous ICD), may serve for monitoring and treating potentially life-threatening arrhythmias of a patient's heart. In the context of this text it shall be understood that the implantable non-transvenous defibrillator device is configured for non-transvenous implantation, that is an implantation such that no electrode leads transvenously are implanted within the heart of a (human or animal) patient. The implantable non-transvenous defibrillator device hence is to be implanted in a patient such that a generator and an electrode arrangement, for example a shock electrode placed on a lead connected to the generator, are implanted extracardially and do not reach into the heart of the patient, that is into the right or left ventricle or the right or left atrium.

In an implantable therapy system of the kind concerned herein, the implantable pacemaker device shall provide for a stimulation in particular if a bradycardia occurs. If a slower-than-normal heart rate is sensed by the pacemaker device, the pacemaker device generates a pacing signal which stimulates cardiac activity such that a suitable heart rate is maintained. The implantable non-transvenous defibrillator device, in turn, shall provide a therapy for a potential tachycardia. If a faster-than-normal heart rate is sensed, such as an atrial or ventricular fibrillation, the defibrillator device emits a shock pulse and in this way resets the heart rhythm to its normal, regular pattern.

Generally, the implantable non-transvenous defibrillator device senses cardiac signals with an arrangement of sensor electrodes outside the heart. Based on sensed signals, herein, the defibrillator device determines whether the emission of a shock pulse is required in order to provide for a defibrillation. As, within the therapy system, the pacemaker device likewise senses signals and (potentially) provides for a therapy action, there is a need that the implantable non-transvenous defibrillator device is enabled, in the presence of the implantable pacemaker device, to reliably sense a tachycardia in order to determine whether the emission of a shock pulse is required.

For establishing a communication between multiple devices implanted in a patient, approaches exist to create an intra-body network (IBN) linking implanted medical devices with each other such that signals may be exchanged in between the implanted medical devices. For example, EP 2 327 609 B1 describes an acoustic communication link in between implanted medical devices for exchanging information in between the implanted medical devices. The acoustic communication link is established to permit wireless communication between the implanted medical devices, wherein transmission parameters may be adapted, such as a sensitivity and a carrier frequency, in order to improve an existing communication link. US 2010/0022836 A1 discloses multidirectional transmitters for in-body devices, such as implantable devices.

EP 3 348 306 A1 discloses a cardiac pacemaking system, comprising: an implantable leadless pacemaker that is configured to be implanted into a chamber of a patient's heart, and an implantable subcutaneous cardioverter-defibrillator, wherein the leadless pacemaker is configured to apply electrical pacing pulses to the heart, particularly in order to provide antibradycardia pacing, and wherein the subcutaneous cardioverter-defibrillator is configured to apply electrical stimulation to the heart for providing defibrillation of the heart, and wherein the leadless pacemaker is configured to sense the patient's heart rhythm, and wherein the cardiac pacemaking system comprises a communication link between the leadless pacemaker and the subcutaneous cardioverter-defibrillator, wherein the leadless pacemaker is configured to send an output signal to the subcutaneous cardioverter-defibrillator via said communication link in an unidirectional fashion when the leadless pacemaker detects a heart rhythm that requires said electrical stimulation of the subcutaneous cardioverter-defibrillator, and wherein the subcutaneous cardioverter-defibrillator is configured to apply said electrical stimulation to the heart when the subcutaneous cardioverter-defibrillator receives said output signal.

It is an object of the instant invention to provide an implantable therapy system in which an implantable pacemaker device and an implantable non-transvenous defibrillator device are allowed to co-exist reliably.

This object is achieved by means of an implantable therapy system comprising the features of claim 1.

Accordingly, in one aspect, an implantable therapy system for an antibradycardia and antitachycardia stimulation comprises an implantable pacemaker device for emitting a cardiac stimulation signal for achieving an intra-cardiac pacing, and an implantable non-transvenous defibrillator device for emitting a shock pulse for achieving a defibrillation, wherein the implantable pacemaker device is configured to transmit an advertising signal for advertising said cardiac stimulation signal, and wherein the implantable non-transvenous defibrillator device is configured to receive said advertising signal and to modify at least one processing function in response to receiving said advertising signal.

Within the implantable therapy system, the implantable pacemaker device may for example be a leadless pacemaker device which is directly implanted into the heart of the patient, for example into the right ventricle, and which does not comprise leads carrying electrodes. Alternatively, the implantable pacemaker device may be a pacemaker device comprising a generator and one or multiple leads, which are connected to and extend from the generator and are implanted to reach into the heart of the patient, such that one or multiple leads are arranged in the right ventricle, the right atrium, the left ventricle, and/or the left atrium.

Within the implantable therapy system, the implantable pacemaker device is configured to emit cardiac stimulation signals for providing for an intracardiac stimulation. The pacemaker device herein shall provide for a therapy in case a bradycardia is detected in order to pace the patient's heart and to in this way pace the heart rate to a desired, normal heart rate.

In turn, the implantable non-transvenous defibrillator device, which is to be implanted outside of the heart and does not comprise leads reaching into the heart, shall provide for a therapy in case a tachycardia is sensed. The implantable non-transvenous defibrillator device herein generally comprises a sensing circuitry such that the implantable non-transvenous defibrillator device may sense signals, may process the sensed signals and, based on the processing, may determine whether it may be required to emit a shock pulse in order to reset a heart rate faster than normal back to a normal heart rate.

As the implantable non-transvenous defibrillator device, beneficially, comprises sensing circuitry and based on its own processing determines the necessity for emitting a shock pulse, it may be the case that sensed signals as processed by the implantable non-transvenous defibrillator device are influenced by stimulation signals emitted by the implantable pacemaker device. In order to avoid a false detection of a tachycardia, herein, the implantable non-transvenous defibrillator device is configured to receive and process an advertising signal, which is generated and transmitted by the implantable pacemaker device in order to advertise a (subsequent) stimulation signal. Hence, by receiving the advertising signal from the implantable pacemaker device, the implantable non-transvenous defibrillator device receives information with respect to a cardiac stimulation signal which is to be emitted by the implantable pacemaker device, such that the implantable non-transvenous defibrillator device may suitably modify its operation by modifying one or multiple processing functions of the implantable non-transvenous defibrillator device.

The processing function of the implantable non-transvenous defibrillator device in particular may relate to the emission of a shock pulse. The modification of the processing function may include a temporary halting of the processing function, or an adaption of the processing function in response to receiving the advertising signal. In particular, the processing function may be modified in response to receiving the advertising signal such that the emission of the cardiac stimulation signal by the implantable pacemaker device does not influence operation of the implantable non-transvenous defibrillator device, in particular to avoid a false detection of a tachycardia and, thus, a false emission of a shock pulse in case a tachycardia is detected erroneously.

In one embodiment, the implantable non-transvenous defibrillator device comprises a lead to be implanted extracardially and a shock electrode arranged on the lead. The shock electrode herein, in an implanted state of the defibrillator device, is placed outside of the heart of the patient, for example in the region of the sternum of the patient, such that a shock pulse for achieving a defibrillation is generated outside of the heart.

Generally, the implantable non-transvenous defibrillator device does not comprise any portions which extend transvenously into the heart, but the defibrillator device is configured to achieve a sensing and emission of signals outside of the heart.

In one embodiment, the implantable pacemaker device is configured to transmit the advertising signal prior to emitting a cardiac stimulation signal. If a cardiac stimulation signal is to be emitted, the implantable pacemaker device announces the emission of the cardiac stimulation signal by sending an advertising signal towards the implantable non-transvenous defibrillator device. The implantable non-transvenous defibrillator device hence is alerted of the imminent emission of the cardiac stimulation signal by means of the implantable pacemaker device, such that the implantable non-transvenous defibrillator device may modify one or multiple processing functions in order to avoid a disturbance of the processing functions by the emission of the cardiac stimulation signal of the implantable pacemaker device.

In one embodiment, the implantable pacemaker device is configured to generate the advertising signal such that the advertising signal is indicative of a timing of the cardiac stimulation signal. For example, the advertising signal may be transmitted by the implantable pacemaker device with a predefined timing relation with respect to a subsequent emission of a cardiac stimulation signal, such that the implantable non-transvenous defibrillator device, upon receiving the advertising signal, is informed about the timing of the stimulation signal and, based on the predefined timing between the advertising signal and the subsequent stimulation signal, is enabled to suitably modify its operation.

In another embodiment, the advertising signal may contain an explicit timing information modulated within the signal, which may be read out by the implantable non-transvenous defibrillator device, such that the timing information is obtained with the advertising signal. The timing information herein may relate to a stimulation pulse which the implantable pacemaker device is about to emit, and may indicate a start of the emission of the stimulation pulse. In another embodiment, by means of the timing information the implantable non-transvenous defibrillator device may be informed about a time window within which the stimulation pulse is to be emitted by the implantable pacemaker device. In yet another embodiment, the timing information may relate to a group of multiple stimulation pulses, wherein the timing information may for example define a time window for the emission of the multiple stimulation pulses and may further provide information about the number of stimulation pulses to be emitted by the implantable pacemaker device within the time window.

In one embodiment, the implantable pacemaker device may be configured to generate said advertising signal such that the advertising signal contains information about at least one of a stimulation timing used for emitting one or multiple cardiac stimulation signals, a stimulation channel used for emitting a cardiac stimulation signal, a stimulation energy used for emitting a cardiac stimulation signal, and a status information about the implantable medical device.

Information contained in the advertising signal generally may relate to the emission of a stimulation signal by the implantable pacemaker device, such as a timing information, information about a stimulation channel, or information about a stimulation energy of the stimulation signal. Information about the stimulation channel may for example indicate whether the stimulation signal is to be emitted into the right or left atrium or the right or left ventricle.

In other embodiments, information contained in the advertising signal may relate to other information not related to the emission of stimulation signals, such as a battery status of the implantable pacemaker device.

In one embodiment, the implantable pacemaker device comprises a signal generation path for generating the cardiac stimulation signal. Within the signal generation path for example suitable generation circuitry may be used to generate the stimulation signal with a desired stimulation energy for emission using an electrode arrangement placed within the heart.

Herein, in one embodiment, the implantable pacemaker device is configured to generate the advertising signal using the (same) signal generation path. The advertising signal hence is generated and transmitted together with the cardiac stimulation signal using beneficially an electrode arrangement implanted in the heart. The electrode arrangement in particular is implanted within the heart of the patient such that at least one electrode is electrically in contact with tissue within the heart, such that electrical signals may be injected into the tissue. By employing for example a subthreshold communication technique the advertising signal may be generated and transmitted together with the stimulation signal at an energy level below an excitation threshold, such that the advertising signal is transmitted together with the stimulation signal, but does not cause an excitation and stimulation within the heart. The advertising signal however may be detected by the implantable non-transvenous defibrillator device using a sensing circuitry of the implantable non-transvenous defibrillator device such that the sensing signal is received by the implantable non-transvenous defibrillator device and may be processed to enable a modification of one or multiple processing functions in response to receiving the advertising signal.

In one embodiment, the implantable pacemaker device is configured to generate the advertising signal as a coded pulse sequence indicative of a subsequent emission of the stimulation signal. The advertising signal may be transmitted using the signal generation path of the stimulation signal, wherein the advertising signal is defined by a unique, coded pulse sequence, which is identifiable upon reception by the implantable non-transvenous defibrillator device. By using a unique code to define the advertising signal in the signal path of the stimulation signal, the implantable non-transvenous defibrillator device is enabled to reliably identify and discern the advertising signal over stimulation signals emitted by the implantable pacemaker device or intrinsic cardiac signals. The implantable non-transvenous defibrillator device hence is enabled to process the received signal and to detect the advertising signal within a sensed signal, such that the implantable non-transvenous defibrillator device is reliably informed about the emission of a stimulation signal by the pacemaker device and may, accordingly, adapt its operation.

In another embodiment, the implantable pacemaker device is configured to transmit the advertising signal using a communication circuitry separate from the signal generation path. In that the advertising signal is sent in a separate path from the stimulation signal, a different communication technology separate from the emission of the stimulation signal is employed, using communication circuitry of the implantable pacemaker device which is separate from the signal generation path of the stimulation signal.

The implantable pacemaker device in particular may comprise communication circuitry configured to establish a communication to other implantable medical devices or to devices external to a patient involving the transmission and reception of acoustic, RF or magnetic signals. For example, the communication technique may use the MICS protocol using a frequency band in between 401 to 406 MHz. In another embodiment, an inductive telemetry technique may be used. In yet another embodiment, a communication based on an induced change of impedances may be used. In yet another embodiment, an intra-body communication network technique, involving a capacitive coupling, a galvanic coupling, an inductive coupling or an RF coupling, may be employed.

In one embodiment, the implantable non-transvenous defibrillator device comprises one or multiple sensing electrodes for sensing a cardiac signal, and control circuitry for processing a sensed cardiac signal. The implantable non-transvenous defibrillator device herein, in one embodiment, is configured to modify the processing of the sensed cardiac signal in response to receiving the advertising signal, in particular in such a way that a stimulation signal emitted by the pacemaker device is not erroneously identified, by the implantable non-transvenous defibrillator device, as a tachycardia, which may otherwise lead to a false emission of a shock pulse.

Different ways to modify a processing function are conceivable within the implantable non-transvenous defibrillator device.

In one embodiment, the implantable non-transvenous defibrillator device may halt a processing, such that in case of an emission of a stimulation signal by the pacemaker device no emission of a shock pulse may occur. The implantable non-transvenous defibrillator device hence resumes its regular operation for sensing and processing cardiac signals in order to identify the necessity of generating and emitting a shock pulse only after a stimulation action by the pacemaker device is complete.

In another embodiment, the implantable non-transvenous defibrillator device may adapt a processing operation in order to filter out signals relating to a stimulation action by the pacemaker device within sensed signals as sensed by the implantable non-transvenous defibrillator device.

For this, the implantable non-transvenous defibrillator device may for example be configured to set a blanking window in order to blank a portion of a sensed cardiac signal in a processing path for processing the sensed cardiac signal. By means of the blanking such portion of the sensed cardiac signal may be canceled which may be influenced by the stimulation signal emitted by the implantable pacemaker device. Hence, a portion of the sensed cardiac signal is blanked out and not included in the processing, such that a therapeutic action of the implantable non-transvenous defibrillator device is not influenced by a stimulation signal as emitted by the pacemaker device.

The blanking window defines which portion of a sensed cardiac signal is blanked out. The blanking herein may take place at different stages within the processing path, for example at an analog stage (analog blanking), at a filter stage, or at a digital stage (digital blanking).

In another embodiment, upon reception of the advertising signal the implantable non-transvenous defibrillator device may initiate a statistical recording or a recording of electrocardiogram signals.

In another aspect, a method for operating an implantable therapy system for an antibradycardia and antitachycardia stimulation comprises: providing an implantable pacemaker device for emitting a cardiac stimulation signal for achieving an intra-cardiac pacing, providing an implantable non-transvenous defibrillator device for emitting a shock pulse for achieving a defibrillation, transmitting, using the implantable pacemaker device, an advertising signal for advertising said cardiac stimulation signal, receiving, using the implantable non-transvenous defibrillator device, said advertising signal, and modifying, using the implantable non-transvenous defibrillator device, at least one processing function in response to receiving said advertising signal.

The advantages and advantageous embodiments described above for the implantable therapy system equally apply also to the method, such that it shall be referred to the above in this respect.

The idea of the invention shall subsequently be described in more detail with reference to the embodiments as shown in the drawings. Herein:
- Fig. 1: shows a schematic drawing of a therapy system of medical devices implanted in a patient;
- Fig. 2: shows a schematic drawing of two medical devices in between which a communication link for a data communication shall be established;
- Fig. 3: shows an embodiment of an implantable therapy system comprising an implantable pacemaker device and an implantable non-transvenous defibrillator device;
- Fig. 4: shows a signal generated by the implantable pacemaker device within the context of a stimulation therapy; and
- Fig. 5: shows a schematic drawing of a processing circuitry of the implantable non-transvenous defibrillator device for processing sensed signals.

Subsequently, embodiments of the invention shall be described in detail with reference to the drawings. In the drawings, like reference numerals designate like structural elements.

It is to be noted that the embodiments are not limiting for the invention, but merely represent illustrative examples.

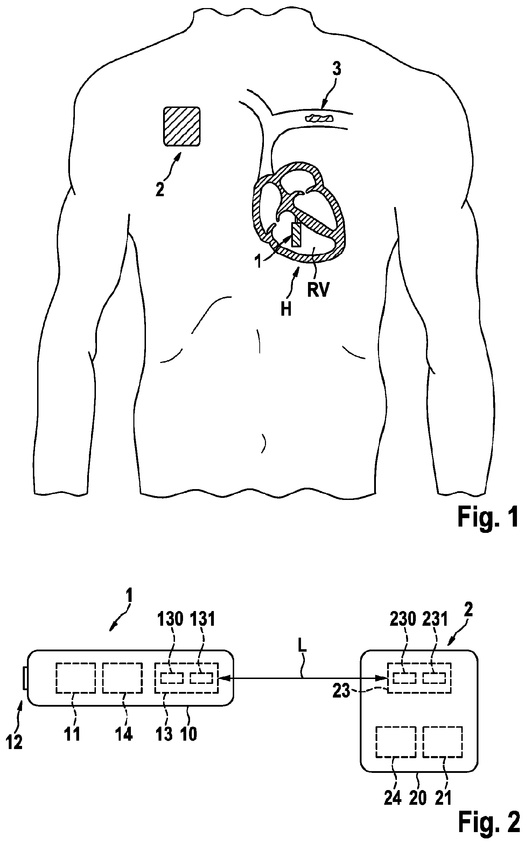

Referring to Fig. 1, within an implantable therapy system implantable medical devices 1, 2, 3 may be implanted in a patient at different locations in order to provide different functions within the patient. For example, a medical device 1 such as a leadless pacemaker device may be implanted e.g. in the right ventricle RV of the patient's heart in order to provide for a pacing action within the heart H. Another medical device 2, for example an implantable non-transvenous defibrillator device, also denoted as non-transvenous implantable cardioverter defibrillator (in short non-transvenous ICD), may subcutaneously be implanted within the chest. Yet another implantable medical device 3 may be for example be a loop recorder or a sensor device, such as a pressure sensor, a flow sensor or a temperature sensor or the like, and may be implanted for example in a blood vessel in order to e.g. sense characteristic parameters such as a blood pressure or a blood flow.

In an implanted state within the patient, the implantable medical devices 1, 2, 3 may together form a therapy system for providing for a cardiac therapy, in particular an antibradycardia and antitachycardia stimulation. In this respect, an implantable pacemaker device 1 may provide for an intracardiac stimulation in order to provide a bradycardia therapy. An implantable defibrillator device 2, in turn, may provide for a therapy in case a tachycardia is detected.

Within a therapy system of this kind, the implantable medical devices 1, 2, 3 may in principle function in an autarkic manner and may hence operate independent of one another. In particular, an implantable pacemaker device 1 may comprise sensing and stimulation circuitry in order to sense cardiac signals and, based on a processing of the cardiac signals, to generate and emit stimulation signals for achieving a therapeutic action. An implantable defibrillator device 2 as well may comprise sensing and stimulation circuitry in order to (independent of the implantable pacemaker device 1) sense cardiac signals in order to process the cardiac signals and to generate and emit a shock pulse in case a defibrillation action is required.

Within a therapy system, it may be desirous to allow a communication in between the medical devices 1, 2, 3. Herein, approaches exist to establish a communication in a wireless fashion by establishing an intra-body network linking the medical devices 1, 2, 3 together, such that data may be exchanged in between the medical device 1, 2, 3. A loop recorder may hence for example record sensor data of a sensor device, or data of a pacemaker or a cardioverter defibrillator, and may also provide data for example to a pacemaker or a cardioverter defibrillator to control a therapeutic action. Signals herein are exchanged in a modulated fashion making use of a particular transmission technology, such as an acoustic, radio frequency (RF) or magnetic (inductive) signal transmission, and a particular modulation scheme, such as a PCM, FSK, PSK, QPSK, FM, or AM modulation or the like.

Referring now to Fig. 2, medical devices 1, 2 to be implanted in a patient may have a small built and may be designed for a low power consumption in order to remain in a patient over a long-term. A first medical device 1, such as an implantable pacemaker device, may herein comprise a housing 10, a control circuitry 11, an electrode arrangement 12 for emitting stimulation signals or receiving sense signals, a communication circuitry 13 and an energy storage 14, for example in the shape of a battery. A second medical device 2, such as an implantable non-transvenous defibrillator device, comprises a housing 20, a control circuitry 21, a communication circuitry 23, and an energy storage 24, for example in the shape of a battery.

The communication circuitry 13, 23, in each case, may comprise a transmission unit 130, 230, and a reception unit 131, 231. The communication circuitry 13, 23, is designed for the specific transmission technology used to establish a communication link L, that is for transmitting and receiving of e.g. acoustic, radio frequency (RF) or magnetic signals. Also, the communication circuitry 13, 23 is for example designed to modulate respectively demodulate signals for transmission and reception, to optimize transmission parameters, to amplify received signals and to process signals in order to forward processed signals to the control circuitry 11, 21 for an analysis and control of the operation of the medical device 1, 2.

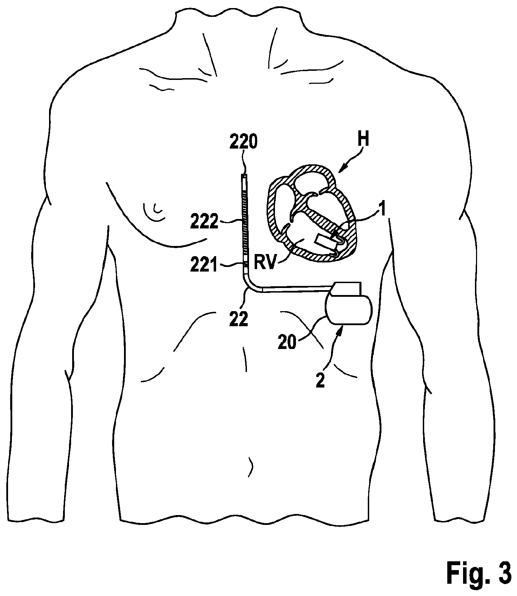

Referring now to Fig. 3, in a specific setup of a therapy system an implantable medical device configured as a leadless pacemaker device 1 is implanted in the heart H of a (human or animal) patient. The leadless pacemaker device 1 is implanted directly into the heart H, for example in the right ventricle RV, in order to couple to tissue within the heart H and to in this way sense cardiac signals within the heart and emit stimulation signals into cardiac tissue.

In addition, an implantable non-transvenous defibrillator device 2 is implanted such that the defibrillator device 2 is completely external to the heart H, the implantable non-transvenous defibrillator device 2 comprising a generator encapsulated within a housing 20, and a lead 22 connected to the housing 20 and carrying sensing electrodes 220, 221 as well as a shock electrode 222 in the shape of a coil formed on a distal portion of the lead 22. The sensing electrodes 220, 221, for example formed as ring electrodes on either side of the shock electrode 222, serve to sense cardiac signals for processing within the generator of the implantable non-transvenous defibrillator device 2, such that based on sensed signals a tachycardia may be identified and a shock pulse may be generated for providing for an antitachycardia therapy.

In a setup as shown in Fig. 3, the pacemaker device 1 implanted within the heart H and the defibrillator device 2 implanted outside of the heart H each comprise sensing circuitry, such that based on a processing of sensed signals in the respective device 1, 2 a therapeutic action may be initiated. Each device 1, 2 herein functions, in principle, independent of the other device 2, 1, such that by means of the pacemaker device 1 implanted in the heart H stimulation signals for overcoming a bradycardia may be generated, whereas by means of the defibrillator device 2 a shock pulse may be emitted for resetting a tachycardic heart rate to a normal heart rate.

If a sensing and processing of sensed signals takes place in the implantable non-transvenous defibrillator device 2 independent of and without knowledge about operation of the pacemaker device 1, an emission of an intracardiac stimulation signal by the pacemaker device 1 may give rise to artifacts in sensed signals as obtained by the defibrillator device 2, potentially causing a detection of a tachycardia based on stimulated signals by the pacemaker device 1. This needs to be avoided in order to ensure a reliable detection and processing of sensed signals by the defibrillator device 2.

For this reason, it herein is proposed that the pacemaker device 1 is configured to transmit an advertising signal for advertising a cardiac stimulation signal, such that the defibrillator device 2 obtains information about a stimulation signal to be emitted by the pacemaker device 1. The defibrillator device 2 is configured to receive the advertising signal, to process the advertising signal and to modify a processing function in response to receiving the advertising signal. In particular, the defibrillator device 2 may be configured to modify one or multiple processing functions such that an unwanted influence of a stimulation signal on a sensing and processing of cardiac signals by the defibrillator device 2 is avoided.

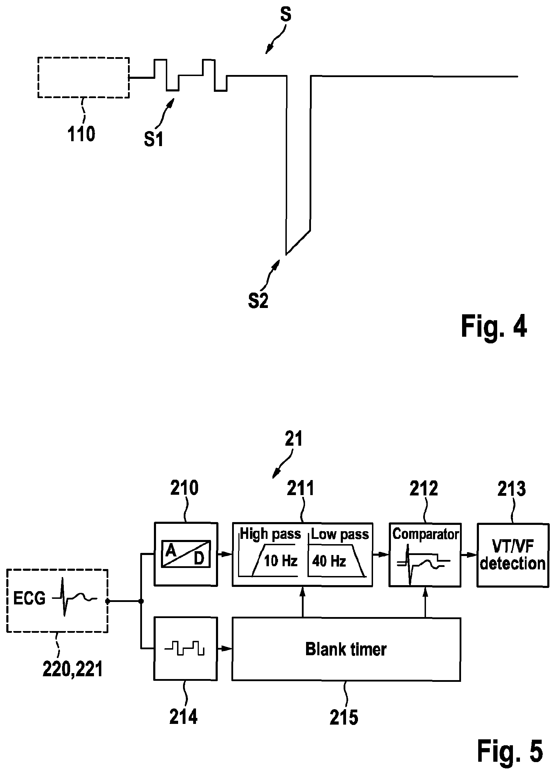

Referring now to Fig. 4, in one embodiment the implantable pacemaker device 1 comprises a signal generation path 110 comprising circuitry for generating a simulation signal S2 for achieving an intracardiac stimulation. The signal generation path 110 uses an electrode arrangement 12 (see Fig. 2) comprising an arrangement of electrodes which are configured to establish a coupling to tissue within the patient's heart, such that stimulation energy may be injected into tissue in order to trigger a pacing action within the heart H.

As shown in Fig. 4, in one embodiment the pacemaker device 1 may establish a communication with the defibrillator device 2 using the same signal generation path 110 which is used to generate the stimulation signal S2. In particular, the pacemaker device 1 in cooperation with the defibrillator device 2 may employ a subthreshold communication technology in which the pacemaker device 1 generates and emits an advertising signal S1 having such a low energy content within an overall transmit signal S such that the advertising signal S1 does not give rise to an excitation and stimulation of tissue within the heart H.

As visible from Fig. 4, the advertising signal S1, in one embodiment, is sent within the transmit signal S prior to the stimulation signal S2, for example at a predefined timing, such that by means of the advertising signal S1 the implantable defibrillator device 2 is informed about the subsequent stimulation signal S2. For example, the stimulation signal S2 may follow the advertising signal S1 by a predefined time distance, such that upon reception of the advertising signal S1 the defibrillator device 2 is informed about the subsequent emission of the stimulation signal S2 and, based on the reception time, is enabled to derive the time at which the stimulation signal S2 will be emitted by the pacemaker device 1.

As visible from Fig. 4, the advertising signal S1 may have the shape of a pulse sequence, wherein the pulse sequence may be formed according to a unique, predefined code, which may be discerned and identified by the defibrillator device 2 in order to detect the advertising signal S1 within a sensed signal as received by the defibrillator device 2.

Upon receiving the advertising signal S1, the defibrillator device 2 may modify its operation, for example by halting or in another way modifying a sensing function. For example, if an advertising signal S1 announcing a subsequent emission of a stimulation signal S2 by the pacemaker device 1 is received at the defibrillator device 2, a generation and emission of a shock pulse by means of the defibrillator device 2 may be prevented for a predefined time span, such that the defibrillator device 2 does not produce a shock pulse at the same time as or close to a stimulation signal as produced by the pacemaker device 1.

The advertising signal S1 may have the shape of a simple warning signal, which may be received by the defibrillator device 2 such that the defibrillator device 2, upon reception of the advertising signal S1, is enabled to modify its operation, the advertising signal S1 as such however not carrying any further information embedded therein.

In another embodiment the advertising signal S1 may contain information encoded within the advertising signal S1, the defibrillator device 2 being enabled to read out and process the information contained in the advertising signal S1. Such information may for example relate to a specific timing of a subsequent stimulation signal S2, to a number of stimulation signals S2 to be emitted within a group of multiple simulation signals, to a time window within which stimulation signals S2 are to be emitted, to a stimulation energy of a stimulation signal S2, and/or to a stimulation channel used for emitting a simulation signal S2. In addition or alternatively, information of the advertising signal S1 may relate to a status of the pacemaker device 1, such as a battery status.

The advertising signal S1 may be sent using the same signal generation path 110 which is used to generate the stimulation signal S2, as shown in Fig. 4. In other embodiments other communication technology separate from the signal generation path 110 may be used, for example an acoustic, capacitive, RF or magnetic (inductive) communication technology, for example within an intra-body communication scheme.

Referring now to Fig. 5, in one embodiment the implantable non-transvenous defibrillator device 2 uses the arrangement of sensing electrodes 220, 221 on the lead 22 for sensing an electrocardiogram signal. The sensed signal is processed by digitizing the signal in an analog-to-digital converter 210, by filtering the digitized signal in a multi-stage filter unit 211, by forwarding the filtered signal to a comparator 212 and by identifying, based on the output of the comparator 212, a sequence of cardiac events using a detection and classification unit 213. Based on the sequence of cardiac events a heart rhythm is identified, and based on a processing a tachycardia or other arrhythmias are classified.

In addition, in a parallel processing path, in a signal identification unit 214 the advertising signal S1 is identified and processed, for example within the signal as sensed by the sensing electrodes 220, 221. Based on timing information obtained by the reception of the advertising signal S1, a timing of a blanking window is set within a timer unit 215, the blanking window providing for a blanking for example within the filter unit 211 and/or within the comparator 212.

By means of the blanking a signal portion of the sensed signal is blanked out and hence suppressed during processing, the blanking window being set such that that signal portion is canceled which may (potentially) be impacted by the stimulation signal S2 as emitted by the pacemaker device 1.

By blanking out a signal portion it is avoided that a sensing and processing at the defibrillator device 2 is disturbed by a stimulation action of the pacemaker device 1. It hence is avoided that the defibrillator device 2 erroneously identifies a stimulation caused by the pacemaker device 1 as a tachycardia or another cardiac arrhythmia, hence preventing a false generation of a shock pulse in response to an erroneous identification of a cardiac arrhythmia.

Hence, based on an advertising signal S1 as produced by and received from the pacemaker device 1, the defibrillator device 2 is enabled to temporarily modify its defibrillation function. It hence is ensured that the pacemaker device 1 and the defibrillator device 2 may function together and may co-exist, although in principle the pacemaker device 1 and the defibrillator device 2 operate independently of one another.

The idea of the invention is not limited to the embodiments described above, but may be implemented in an entirely different fashion.

The pacemaker device may be a leadless pacemaker device, or may be a pacemaker device having one or multiple leads. A pacemaker device having one or multiple leads for example comprises a generator which is to be implanted subcutaneously in a patient, the one or multiple leads carrying one or multiple electrodes extending from the generator and being implanted into the heart for sensing signals and for providing a stimulation signal within the heart.

### List of reference numerals

- 1: Pacemaker device
- 10: Housing
- 11: Control circuitry
- 110: Signal generation path
- 12: Electrode arrangement
- 13: Communication circuitry
- 130: Transmission unit
- 131: Reception unit
- 14: Energy storage
- 2: Non-transvenous defibrillator device
- 20: Housing
- 21: Control circuitry
- 210: Analog-to-digital converter
- 211: Filter unit
- 212: Comparator
- 213: Detection unit
- 214: Signal identification unit
- 215: Timer unit
- 22: Lead
- 220, 221: Sensing electrode
- 222: Shock electrode
- 23: Communication circuitry
- 230: Transmission unit
- 231: Reception unit
- 24: Energy storage
- 3: Implantable medical device
- H: Heart
- L: Communication link
- RV: Right ventricle
- S: Signal
- S1: Advertising signal
- S2: Stimulation signal

## Claims

1. An implantable therapy system for an antibradycardia and antitachycardia stimulation, comprising:
an implantable pacemaker device (1) for emitting a cardiac stimulation signal (S2) for achieving an intra-cardiac pacing, and
an implantable non-transvenous defibrillator device (2) for emitting a shock pulse for achieving a defibrillation,
wherein the implantable pacemaker device (1) is configured to transmit an advertising signal (S1) for advertising said cardiac stimulation signal (S2), and
wherein the implantable non-transvenous defibrillator device (2) is configured to receive said advertising signal (S1) and to modify at least one processing function in response to receiving said advertising signal (S1).

2. The implantable therapy system according to claim 1, wherein the implantable pacemaker device (1) is a leadless pacemaker device.

3. The implantable therapy system according to claim 1 or 2, wherein the implantable non-transvenous defibrillator device (2) comprises a lead (22) to be implanted extracardially and a shock electrode (222) arranged on the lead (22).

4. The implantable therapy system according to one of claims 1 to 3, wherein the implantable pacemaker device (1) is configured to transmit said advertising signal (S1) prior to emitting said cardiac stimulation signal (S2).

5. The implantable therapy system according to one of the preceding claims, wherein the implantable pacemaker device (1) is configured to generate said advertising signal (S1) such that said advertising signal (S1) is indicative of a timing of said cardiac stimulation signal (S2).

6. The implantable therapy system according to one of the preceding claims, wherein the implantable pacemaker device (1) is configured to generate said advertising signal (S1) such that said advertising signal (S1) contains information with respect to at least one of a stimulation timing used for emitting said cardiac stimulation signal (S2), a stimulation channel used for emitting said cardiac stimulation signal (S2), a stimulation energy used for emitting said cardiac stimulation signal (S2), and a status information of the implantable pacemaker device (1).

7. The implantable therapy system according to one of the preceding claims, wherein the implantable pacemaker device (1) comprises a signal generation path (110) for generating said cardiac stimulation signal (S2).

8. The implantable therapy system according to claim 7, wherein the implantable pacemaker device (1) is configured to generate the advertising signal (S1) using the signal generation path (110).

9. The implantable therapy system according to claim 8, wherein the implantable pacemaker device (1) comprises an electrode arrangement (12) for emitting said cardiac stimulation signal (S2) and for transmitting said advertising signal (S1) using the signal generation path (110).

10. The implantable therapy system according to one of claims 7 to 9, wherein the implantable pacemaker device (1) is configured to transmit the advertising signal (S1) using a sub-threshold communication technique.

11. The implantable therapy system according to one of claims 7 to 10, wherein the implantable pacemaker device (1) is configured to generate said advertising signal (S1) as a coded pulse sequence indicative of a subsequent emission of said stimulation signal (S2).

12. The implantable therapy system according to claim 7, wherein the implantable pacemaker device (1) is configured to transmit the advertising signal (S1) using a communication circuitry (13) separate from said signal generation path (110).

13. The implantable therapy system according to one of the preceding claims, wherein the implantable non-transvenous defibrillator device (2) comprises at least one sensing electrode (220, 221) for sensing a cardiac signal and control circuitry (21) for processing a sensed cardiac signal, wherein the implantable non-transvenous defibrillator device (2) is configured, for modifying said at least one processing function, to modify said processing of said sensed cardiac signal in response to receiving said advertising signal (S1).

14. The implantable therapy system according to claim 13, wherein the implantable non-transvenous defibrillator device (2) is configured to set a blanking window in order to blank a portion of said sensed cardiac signal in a processing path for processing said sensed cardiac signal.

## Patentansprüche

1. Implantierbares Therapiesystem für eine Antibradykardie- und Antitachykardiestimulation, umfassend:
eine implantierbare Schrittmachervorrichtung (1) zum Emittieren eines kardialen Stimulationssignals (S2) zum Erreichen einer intrakardialen Stimulierung, und
eine implantierbare, nicht transvenöse Defibrillatorvorrichtung (2) zum Emittieren eines Schockimpulses zum Erreichen einer Defibrillation,
wobei die implantierbare Schrittmachervorrichtung (1) dazu konfiguriert ist, ein Ankündigungssignal (S1) zu senden, um das kardiale Stimulationssignal (S2) anzukündigen, und
wobei die implantierbare nicht transvenöse Defibrillatorvorrichtung (2) dazu konfiguriert ist, das Ankündigungssignal (S1) zu empfangen und in Reaktion auf das Empfangen des Ankündigungssignals (S1) mindestens eine Verarbeitungsfunktion zu modifizieren.

2. Implantierbares Therapiesystem nach Anspruch 1, wobei die implantierbare Schrittmachervorrichtung (1) eine kabellose Schrittmachervorrichtung ist.

3. Implantierbares Therapiesystem nach Anspruch 1 oder 2, wobei die implantierbare nicht transvenöse Defibrillatorvorrichtung (2) ein extrakardial zu implantierendes Kabel (22) und eine an dem Kabel (22) angeordnete Schockelektrode (222) umfasst.

4. Implantierbares Therapiesystem nach einem der Ansprüche 1 bis 3, wobei die implantierbare Schrittmachervorrichtung (1) dazu konfiguriert ist, das Ankündigungssignal (S1) vor dem Emittieren des kardialen Stimulationssignals (S2) zu senden.

5. Implantierbares Therapiesystem nach einem der vorhergehenden Ansprüche, wobei die implantierbare Schrittmachervorrichtung (1) dazu konfiguriert ist, das Ankündigungssignal (S1) so zu erzeugen, dass das Ankündigungssignal bezeichnend für eine Zeitsteuerung des kardialen Stimulationssignals (S2) ist.

6. Implantierbares Therapiesystem nach einem der vorhergehenden Ansprüche, wobei die implantierbare Schrittmachervorrichtung (1) dazu konfiguriert ist, das Ankündigungssignal (S1) so zu erzeugen, dass das Ankündigungssignal (S1) Informationen in Bezug auf wenigstens eine/n von einer Stimulationszeitsteuerung, verwendet zum Emittieren des kardialen Stimulationssignals (S2), einen Stimulationskanal, verwendet zum Emittieren des kardialen Stimulationssignals (S2), eine Stimulationsenergie, verwendet zum Emittieren des kardialen Stimulationssignals (S2), und eine Statusinformation der implantierbaren Schrittmachervorrichtung (1) umfasst.

7. Implantierbares Therapiesystem nach einem der vorhergehenden Ansprüche, wobei die implantierbare Schrittmachervorrichtung (1) einen Signalerzeugungspfad (110) zum Erzeugen des kardialen Stimulationssignals (S2) umfasst.

8. Implantierbares Therapiesystem nach Anspruch 7, wobei die implantierbare Schrittmachervorrichtung (1) dazu konfiguriert ist, das Ankündigungssignal (S1) unter Verwendung des Signalerzeugungspfads (110) zu erzeugen.

9. Implantierbares Therapiesystem nach Anspruch 8, wobei die implantierbare Schrittmachervorrichtung (1) eine Elektrodenanordnung (12) zum Emittieren des kardialen Stimulationssignals (S2) und zum Senden des Ankündigungssignals (S1) unter Verwendung des Signalerzeugungspfads (110) umfasst.

10. Implantierbares Therapiesystem nach einem der Ansprüche 7 bis 9, wobei die implantierbare Schrittmachervorrichtung (1) dazu konfiguriert ist, das Ankündigungssignal (S1) unter Verwendung einer Technik der unterschwelligen Kommunikation zu senden.

11. Implantierbares Therapiesystem nach einem der Ansprüche 7 bis 10, wobei die implantierbare Schrittmachervorrichtung (1) dazu konfiguriert ist, das Ankündigungssignal (S1) als eine codierte Impulsfolge, bezeichnend für eine nachfolgende Emission des Stimulationssignals (S2), zu senden.

12. Implantierbares Therapiesystem nach Anspruch 7, wobei die implantierbare Schrittmachervorrichtung (1) dazu konfiguriert ist, das Ankündigungssignal (S1) unter Verwendung einer vom Signalerzeugungspfad (110) separaten Kommunikationsschaltung (13) zu erzeugen.

13. Implantierbares Therapiesystem nach einem der vorhergehenden Ansprüche, wobei die implantierbare nicht transvenöse Defibrillatorvorrichtung (2) mindestens eine Messelektrode (220, 221) zum Messen eines kardialen Signals und eine Steuerschaltung (21) zum Verarbeiten eines gemessenen kardialen Signals umfasst, wobei die implantierbare nicht transvenöse Defibrillatorvorrichtung (2) zum Modifizieren der mindestens einen Verarbeitungsfunktion konfiguriert ist, um die Verarbeitung des gemessenen kardialen Signals in Reaktion auf das Empfangen des Ankündigungssignals (S1) zu modifizieren.

14. Implantierbares Therapiesystem nach Anspruch 13, wobei die implantierbare nicht transvenöse Defibrillatorvorrichtung (2) dazu konfiguriert ist, ein Ausblendfenster zu setzen, um einen Teil des gemessenen kardialen Signals in einem Verarbeitungspfad zum Verarbeiten des gemessenen kardialen Signals auszublenden.

## Revendications

1. Système thérapeutique implantable pour une stimulation d'antibradycardie et d'antitachycardie, comprenant :
un dispositif de stimulateur cardiaque implantable (1) destiné à émettre un signal de stimulation cardiaque (S2) pour obtenir une stimulation intracardiaque, et
un dispositif de défibrillateur non transveineux implantable (2) destiné à émettre une impulsion de choc pour obtenir une défibrillation,
dans lequel le dispositif de stimulateur cardiaque implantable (1) est configuré pour transmettre un signal d'annonce (S1) afin d'annoncer ledit signal de stimulation cardiaque (S2), et
dans lequel le dispositif de défibrillateur non transveineux implantable (2) est configuré pour recevoir ledit signal d'annonce (S1) et pour modifier au moins une fonction de traitement en réponse à la réception dudit signal d'annonce (S1).

2. Système thérapeutique implantable selon la revendication 1, dans lequel le dispositif de stimulateur cardiaque implantable (1) est un dispositif de stimulateur cardiaque sans câbles.

3. Système thérapeutique implantable selon la revendication 1 ou 2, dans lequel le dispositif de défibrillateur non transveineux implantable (2) comprend un câble (22) à implanter de manière extracardiaque et une électrode de choc (222) agencée sur le câble (22).

4. Système thérapeutique implantable selon l'une des revendications 1 à 3, dans lequel le dispositif de stimulateur cardiaque implantable (1) est configuré pour transmettre ledit signal d'annonce (S1) avant d'émettre ledit signal de stimulation cardiaque (S2).

5. Système thérapeutique implantable selon l'une des revendications précédentes, dans lequel le dispositif de stimulateur cardiaque implantable (1) est configuré pour générer ledit signal d'annonce (S1) de telle sorte que ledit signal d'annonce (S1) indique une synchronisation dudit signal de stimulation cardiaque (S2).

6. Système thérapeutique implantable selon l'une des revendications précédentes, dans lequel le dispositif de stimulateur cardiaque implantable (1) est configuré pour générer ledit signal d'annonce (S1) de telle sorte que ledit signal d'annonce (S1) contient des informations concernant au moins un parmi une synchronisation de stimulation utilisée pour émettre ledit signal de stimulation cardiaque (S2), un canal de stimulation utilisé pour émettre ledit signal de stimulation cardiaque (S2), une énergie de stimulation utilisée pour émettre ledit signal de stimulation cardiaque (S2), et une information de statut du dispositif de stimulateur cardiaque implantable (1).

7. Système thérapeutique implantable selon l'une des revendications précédentes, dans lequel le dispositif de stimulateur cardiaque implantable (1) comprend un trajet de génération de signal (110) pour générer ledit signal de stimulation cardiaque (S2).

8. Système thérapeutique implantable selon la revendication 7, dans lequel le dispositif de stimulateur cardiaque implantable (1) est configuré pour générer le signal d'annonce (S1) en utilisant le trajet de génération de signal (110).

9. Système thérapeutique implantable selon la revendication 8, dans lequel le dispositif de stimulateur cardiaque implantable (1) comprend un agencement d'électrodes (12) destiné à émettre ledit signal de stimulation cardiaque (S2) et transmettre ledit signal d'annonce (S1) en utilisant le trajet de génération de signal (110).

10. Système thérapeutique implantable selon l'une des revendications 7 à 9, dans lequel le dispositif de stimulateur cardiaque implantable (1) est configuré pour transmettre le signal d'annonce (S1) en utilisant une technique de communication de type sous-seuil.

11. Système thérapeutique implantable selon l'une des revendications 7 à 10, dans lequel le dispositif de stimulateur cardiaque implantable (1) est configuré pour générer ledit signal d'annonce (S1) en tant que séquence d'impulsions codée indiquant une émission consécutive dudit signal de stimulation (S2).

12. Système thérapeutique implantable selon la revendication 7, dans lequel le dispositif de stimulateur cardiaque implantable (1) est configuré pour transmettre le signal d'annonce (S1) en utilisant un circuit de communication (13) distinct dudit trajet de génération de signal (110).

13. Système thérapeutique implantable selon l'une des revendications précédentes, dans lequel le dispositif de défibrillateur non transveineux implantable (2) comprend au moins une électrode de détection (220, 221) destinée à détecter un signal cardiaque et un circuit de commande (21) destiné à traiter un signal cardiaque détecté, dans lequel le dispositif de défibrillateur non transveineux implantable (2) est configuré, pour la modification de ladite au moins une fonction de traitement, afin de modifier ledit traitement dudit signal cardiaque détecté en réponse à la réception dudit signal d'annonce (S1).

14. Système thérapeutique implantable selon la revendication 13, dans lequel le dispositif de défibrillateur non transveineux implantable (2) est configuré pour définir une fenêtre de détourage afin de détourer une partie dudit signal cardiaque détecté dans un trajet de traitement afin de traiter ledit signal cardiaque détecté.
